# EUROPEAN PATENT SPECIFICATION

(11) **EP 2 709 671 B1**
(45) Date of publication and mention of the grant of the patent: **29.11.2017**
(21) Application number: 12722487.1
(22) Date of filing: 17.05.2012
(51) Int. Cl.: C07K 16/46, C07K 14/725, C07K 14/705, C12N 5/0783

(54) **METHOD**
VERFAHREN
PROCÉDÉ

(30) Priority: 17.05.2011 GB 201108236
(43) Date of publication of application: 26.03.2014
(73) Proprietor: UCL Business PLC, London, W1T 4TP (GB)
(72) Inventor: STAUSS, Hans, London W1T 4TP (GB); THOMAS, Sharyn, London W1T 4TP (GB)
(74) Representative: D Young & Co LLP
(86) International application number: PCT/GB2012/051113
(87) International publication number: WO 2012/156747

(56) References cited:
- WO-A1-2010/025177
- WO-A2-2009/091826
- VERA JUAN F ET AL: "Immunotherapy of Human Cancers Using Gene Modified TLymphocytes", PubMed Central (PMC) - Author Manuscript. CURRENT GENE THERAPY, vol. 9, no. 5 October 2009 (2009-10), pages 1-22, XP002683648, Retrieved from the Internet: URL:http://www.ncbi.nlm.nih.gov/pmc/articl es/PMC2952647/pdf/nihms228210.pdf [retrieved on 2012-09-19]

## Description

### FIELD OF THE INVENTION

The present invention relates to a method for increasing the sensitivity of a T cell expressing a cell-surface antibody. It relates to a method for increasing the ability of such a T cell to recognise low concentrations of target antigen. The antibody may be part of a chimeric antigen receptor (CAR), comprising an extracellular antibody-domain and a cytoplasmic signalling domain.

### BACKGROUND TO THE INVENTION

T-cell-based cancer immunotherapy approaches are commonly based on the capacity of CD8⁺ cytotoxic T cells (CTLs) to recognize and destroy malignant cells. Cancer cells present peptides derived from tumor-associated antigens (TAAs) at their cell surface in conjunction with major histocompatibility complex (MHC) class I molecules. CD4⁺ T cells also play an important role in antitumor immunity. For example, CD4⁺ T cells improve the capacity of dendritic cells (DCs) to induce CTLs by the interaction between CD40 on DCs and CD40 ligand on activated CD4⁺ T cells. Furthermore, CD4⁺ T cells provide help for the maintenance and expansion of CTLs by secreting cytokines such as interleukin-(IL-) 2.

In order to extend the recognition specificity of T lymphocytes beyond their classical MHC-peptide complexes, a gene-therapeutic strategy has been developed that allows redirecting T cells to defined tumour cell surface antigens. This strategy uses both the cellular and humoral arm of the immune response by assembling an antigen-binding moiety, most commonly a single chain variable fragment (scFv); together with a lymphocyte-activating signalling chain, such as the CD3 chain. Once this chimeric antigen receptor (CAR) is expressed at the surface of a T cell, the antigen-binding moiety binds to its antigen and an activating signal is transmitted into the T cell, which in turn triggers its effector functions against the target cell.

The use of CARs to redirect T cells specifically against TAA-expressing tumor cells has a number of theoretical advantages over classical T-cell-based immunotherapies. In contrast to the long-lasting procedure of *in vitro* selection, characterization, and expansion of T-cell clones with native specificity for MHC-tumor peptide complexes, genetic modification of polyclonal T-cell populations allows TAA-specific T cells to be generated in one to two weeks. Engraftment with CARs enables T cells to MHC-independent antigen recognition; thus major immune escape mechanisms of tumors such as downregulation of MHC molecules are efficiently bypassed. Furthermore, proliferation and survival of modified T cells can be improved by the implementation of a multitude of signaling domains from different immune receptors in a single CAR. In addition, T cells can be rendered more resistant against the immunosuppressive milieu in tumor tissue. In addition to cancer immunotherapy, CAR-modified lymphocytes have been successfully applied for the treatment of virus infections, and more recently, first experimental studies have been published using CARs engrafted on regulatory T cells (Tregs) for the treatment of autoimmune diseases.

The first clinical studies using CAR-modified T cells have been reported and a number of clinical trials are currently in progress.

Although CAR technology shows initial promise, improvements are desirable in order to maximise the therapeutic potential of CAR-expressing T cells. In particular, there is a need to improve sensitivity of targeted T cells, so that they can respond to low concentrations of target antigen.

### DESCRIPTION OF THE FIGURES

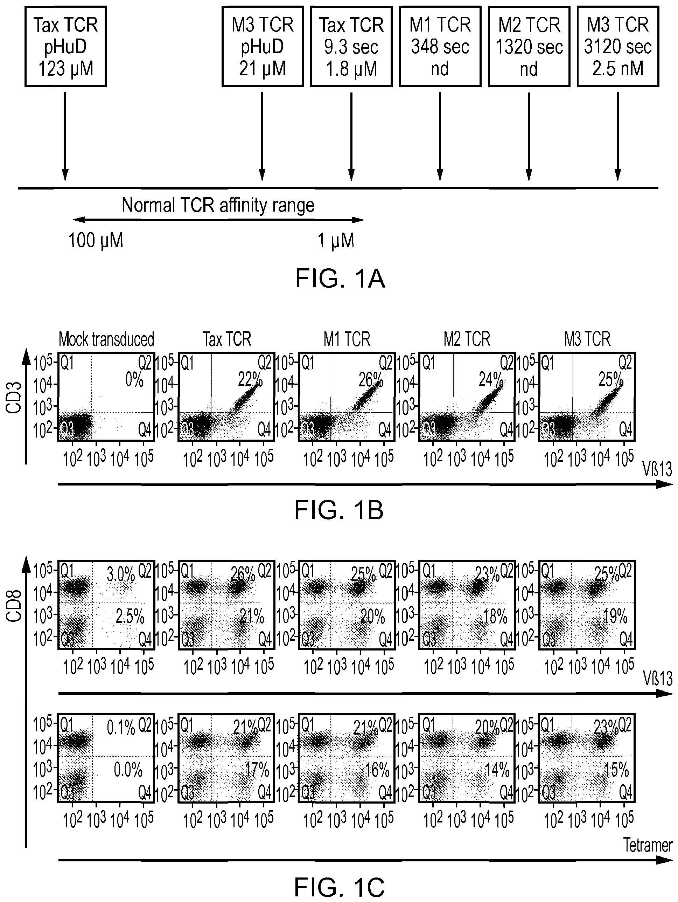

Figure 1: **TCR Affinity Ranges and human Jurkat cells and primary T cells transduced with the wild-type and affinity matured TCR constructs**
   (A) Schematic diagram showing the natural TCR - peptide / MHC affinity range and the affinities of the Tax TCR and affinity matured TCRs to either the pTax peptide or the pHuD peptide and the positions they occupy in the affinity range. Indicated are the dissociation constant K_{D} of the TCR-HLA/peptide binding and the half-life t_{1/2} (in seconds) of the interaction. Jurkat cells (B) or activated primary T cells (C) were transduced with pMP71 vectors encoding the indicated TCR. 72 h after transduction, Jurkat cells were stained with anti-CD3 and Vβ13 antibodies (B) and mock transduced primary T cells or TCR transduced primary T cells were stained with anti-CD3 and CD8 antibodies and either Vβ13 antibodies or tetramer (C). Primary T cells were gated on the CD3+ T cell population. For the Jurkat cell transduction shown is a representative of at least 6 independent experiments, which demonstrated similar results. For the primary T cell transduction shown is a representative of at least 20 independent experiments, which demonstrated similar results.

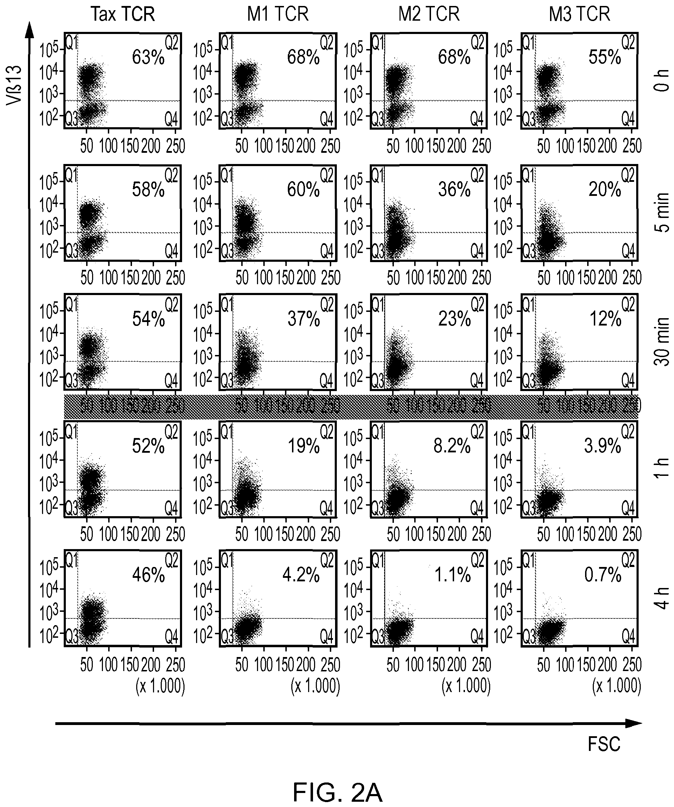

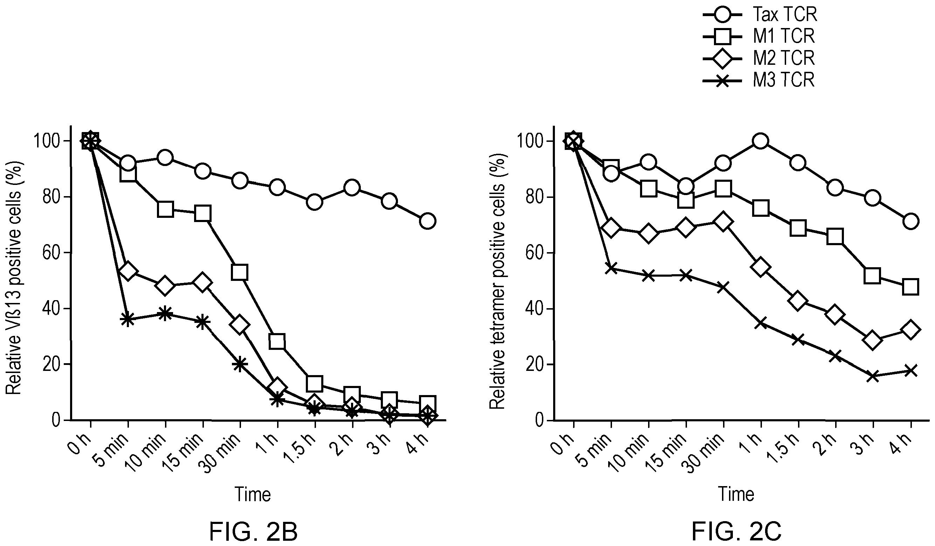

Figure 2: **T cells transduced with affinity matured TCR down-regulate TCR faster and to a greater level than T cells transduced with Tax-TCR.**
   (A, B) Jurkat cells and (C) primary T cells transduced with the Tax TCR and the affinity matured TCR were co-cultured with T2 stimulator cells loaded with saturating concentration of Tax peptide (100 µM) for the stated time periods. Jurkat cells were stained with anti-Vβ13 antibodies and primary T cells were stained with anti-CD8 antibodies and tetramer (the axis of the FACS plots range from 10¹ to 10⁵). Shown is a representative of 4 independent Jurkat experiments, showing similar results. Primary T cells were gated on the CD8+ T cell population; the percentage of tetramer-positive CD8+ T cells at time 0 was 63%, 68%, 68%, 55% for the Tax-TCR, M1, M2, M3-TCR, respectively. Shown is the relative loss of tetramer-positive T cells after peptide stimulation. Shown is a representative of 3 independent experiments showing similar results.

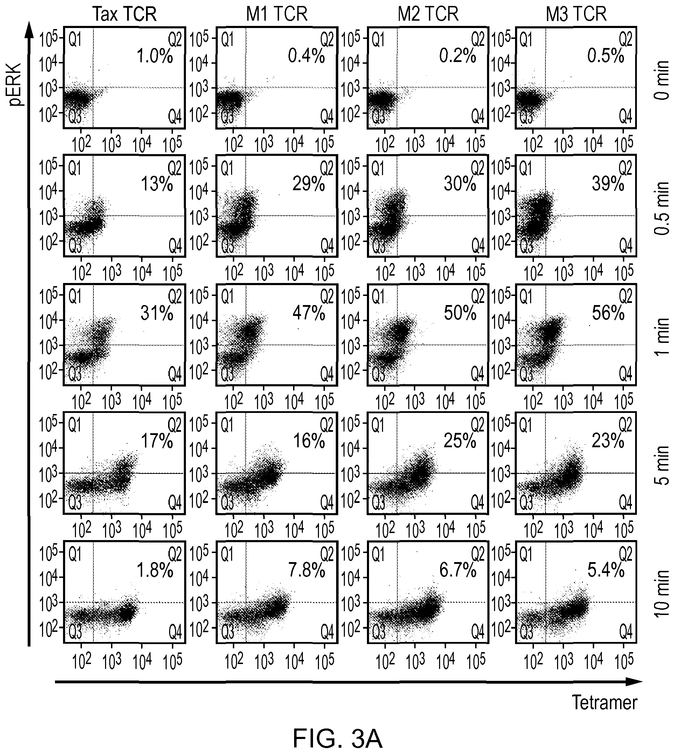

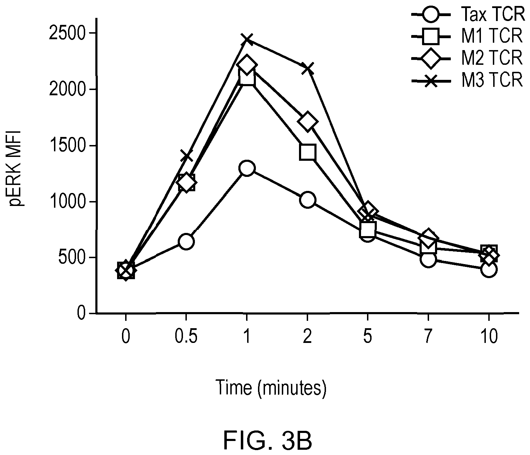

Figure 3: **Up-regulation of phospho-ERK after stimulation of primary T cells transduced with the TCRs constructs and stimulated with pTax/HLA-A2 tetramer**
   (A) Primary T cells transduced with the Tax TCRs and undergone 5 rounds of weekly peptide stimulation had the following percent of CD8+tetramer+ T cells; Tax TCR = 83%, M1 TCR = 88%, M2 TCR = 92% and M3 TCR = 94%. 2 x 10⁵ T cells were stimulated with pTax/MHC tetramer for the stated times, and then immediately fixed, permeabilised and stained with anti-pERK antibodies. (A) FACS plots of tetramer expression and pERK expression at the stated times (the axis of the FACS plots range from 10¹ to 10⁵). (B) Mean fluorescent intensity of pERK at the stated times. These figures are representative of 4 individual experiments that showed similar results.

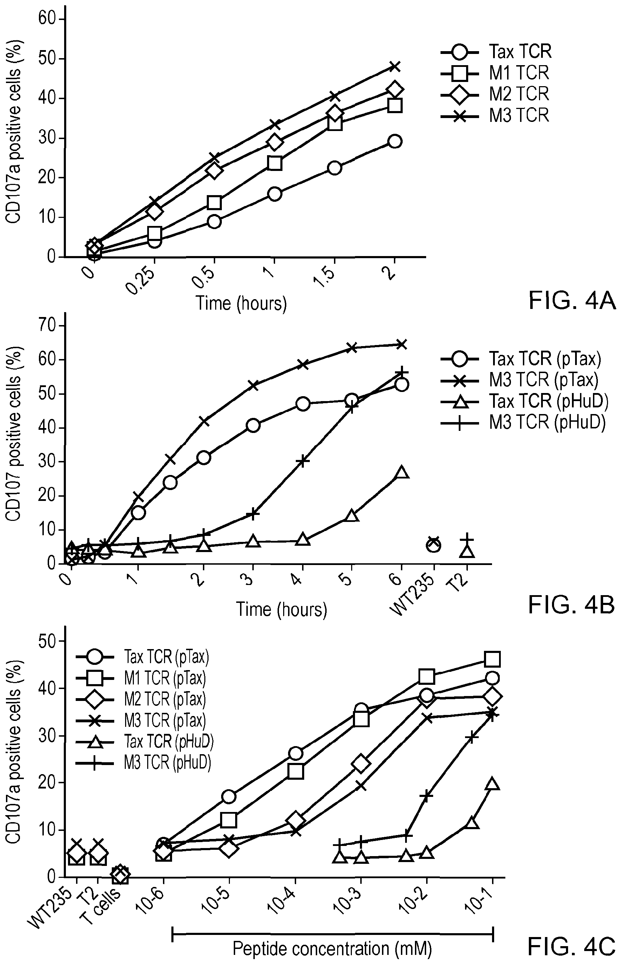

Figure 4: **Kinetics of antigen-specific CD107a up-regulation**
   (A) Primary T cells transduced with the Tax-TCR and affinity matured TCR and undergone 3 rounds of weekly peptide stimulation had the following CD8+ / tetramer+ expression; Tax TCR = 92%, M1 TCR = 96%, M2 TCR = 95% and M3 TCR = 98%. 2 x 10⁵ T cells were stimulated with 2 x 10⁵ T2 cells loaded with saturating concentration of pTax peptide in the presence of CD107a, brefeldin A and GolgiStop for the stated times. (A) Shown are the percentages of CD8+CD107a+ cells as a proportion of the tetramer-positive cells. The Figure is representative of 4 individual experiments that demonstrated similar results. (B) Primary T cells transduced with the Tax-TCR and M3-TCR and undergone 3 rounds of weekly peptide stimulation had the following percentage CD8+tetramer+ T cells; Tax TCR = 98% and M3 TCR = 99%. 2 x 10⁵ T cells were stimulated with 2 x 10⁵ T2 cells loaded with saturating concentration of pTax peptide or pHuD peptide in the presence of CD107a, brefeldin A and GolgiStop for the stated times. (B) Shown are the percentages of CD8+CD107a+ cells as a proportion of the tetramer-positive cells. Stimulation with T2 cells pulsed with the control pWT235 peptide or without peptide did not result in CD107 upregulation. This figure is representative of 3 individual experiments which showed similar results. (C) Primary T cells transduced with the Tax-TCR and affinity matured TCR and undergone 5 rounds of weekly peptide stimulation had the following percentage CD8+tetramer+ T cells; Tax TCR = 91%, M1 TCR = 89%, M2 TCR = 86% and M3 TCR = 86%. 2 x 10⁵ T cells were stimulated with 2 x 10⁵ T2 cells loaded with decreasing concentrations of pTax peptide or pHuD peptide in the presence of CD107a, brefeldin A and Golgi Stop. CD107a expression was determined after a 5 h stimulation period. Stimulation with T2 cells pulsed with the control pWT235 peptide or without peptide did not result in CD107 upregulation. Shown is a representative of 3 individual experiments that showed similar results.

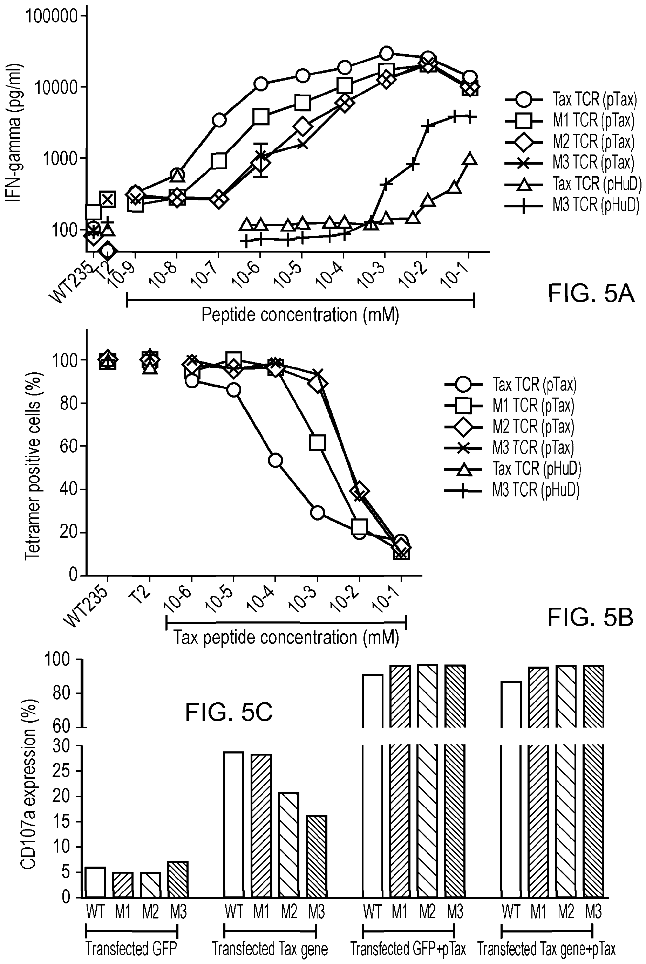

Figure 5: **Primary T cells transduced with the high affinity TCR show lower peptide sensitivity compared to Tax-TCR transduced T cells**
   (A) Freshly transduced primary T cells had the following percentage of CD8+Vβ13+ T cells; Tax TCR = 26%, M1 TCR = 24%, M2 TCR = 25% and M3 TCR = 26%. 1 x 10⁵ bulk transduced T cells were co-cultured with T2 cells at a 1:1 ratio of transduced T cells : T2 cells loaded with pTax peptide or pHuD peptide at the stated concentrations. After 18 h stimulation, the supernatant was removed to determine IFN-gamma release by ELISA. Stimulation with T2 cells pulsed with the control pWT235 peptide or without peptide defined the background level of IFN-gamma release. This is representative of 4 individual experiments that demonstrated similar results. (B) Primary T cells transduced with the Tax TCR and affinity matured versions and undergone 5 rounds of weekly peptide stimulation had the following percentage of CD8+Vβ13+ T cells; Tax TCR = 97%, M1 TCR = 99%, M2 TCR = 99% and M3 TCR = 99%. 2 x 10⁵ T cells were stimulated with 2 x 10⁵ T2 cells loaded with decreasing concentration of pTax peptide. After a 18 h co-culturing period cells were stained with tetramer. Shown is the relative loss of tetramer-positive T cells after peptide stimulation. The experiment is representative of 3 individual experiments that showed similar results. (C) Primary T cells that were transduced with the Tax TCR or the affinity matured version and expanded using 5 rounds of weekly peptide stimulation had the following CD8+/Vβ13 expressing cells; Tax TCR = 89%, M1 TCR = 93%, M2 TCR = 91% and M3 TCR = 90%. 2 x 10⁵ T cells were stimulated with either 2 x 10⁵ HeLa GFP positive cells that had been transduced with either the GFP plasmid or the GFP-Tax gene fusion plasmid or the same transduced HeLa cells that were loaded with 100 µM exogenous pTax peptide. Cells were co-cultured in the presence of CD107a, brefeldin A and GolgiStop for 18 h. Shown are the percentages of CD8+ / CD107a+ T cells. The figure is representative of 5 independent experiments that demonstrated similar results.

### SUMMARY OF ASPECTS OF THE INVENTION

The natural affinity range of antibodies is 1,000-100,000 times higher than the natural affinity of T cell receptors (TCR). The present inventors have surprisingly found that increasing the affinity range of TCR by 1000-fold into the affinity range of antibodies did not improve T cell function, and actually *increased* the concentration of antigen required to trigger T cell activation.

This has major implications in the field of T-cell targeting with antibody constructs, such as chimeric antigen receptors (CARs). In order to achieve optimal T-cell sensitivity, the natural affinity of antibodies needs to be lowered, for example into the natural affinity range of TCRs.

Thus, the present invention provides a method for increasing the sensitivity of a T cell expressing a cell-surface antibody to a target antigen, which comprises the step of lowering the affinity of the antibody to the target antigen by at least 100-fold, wherein the affinity of the antibody is lowered by mutagenesis of the antibody, followed by *in vitro* selection for low affinity variants.

The affinity of the antibody to the target antigen may be lowered by at least 1000-fold.

The affinity of the antibody for the target antigen, following lowering, (i.e. the affinity for the variant antibody) may be in the range of 100 - 1µM.

The variant antibody, following affinity lowering, may have a dissociation half time from the target antigen in the range of 5 - 60 seconds.

The antibody may be part of a chimeric antigen receptor (CAR) comprising:
(i) an antibody domain, expressed at the T cell surface; and
(ii) a cytoplasmic signalling domain.

Described herein is a T cell made by a method according to the first aspect of the invention.

The T cell may express a variant cell surface antibody derived from a starting antibody, wherein the variant cell surface antibody has an affinity for its target antigen which is at least 100-fold less than that of the starting antibody.

The T cell may express a cell-surface antibody having an affinity for the target antigen in the range of 100 - 1 µM.

The T cell may express a cell-surface antibody having a dissociation half-time from the target antigen which is in the range of 5 - 60 seconds.

Described herein is a chimeric antigen receptor (CAR) for expression in a T cell, comprising an antibody domain and a signalling domain, in which the antibody domain has an affinity for a target antigen in the range of 100 -1 µM.

Also described herein is a chimeric antigen receptor (CAR) for expression in a T cell, comprising an antibody domain and a signalling domain, in which the antibody domain binds to a target antigen with a dissociation half time in the range of 5 - 60 seconds.

Further described herein is a nucleic acid sequence encoding a cell-surface antibody as described above. The nucleic acid sequence may, for example, encode a CAR according to the present description.

Described herein is a vector comprising a nucleic acid sequence according to the present description, which is capable of expressing a CAR in a T cell.

Further described herein is a T cell which expresses a CAR according to the present description.

Further described herein is a vector according to the present description, or a T cell according to the present description for use in the treatment of a disease.

Also described herein is a method for treating a disease in a subject, which comprises the step of administering a vector according to the present description, or a T cell according to the present description to the subject.

Also described herein is the use of a vector according to the present description, or a T cell according to the present description in the manufacture of a medicament for use in the treatment of a disease.

The disease may be cancer, viral infection or autoimmunity.

### DETAILED DESCRIPTION

### SENSITIVITY

The present invention relates to a method for increasing the sensitivity of a T cell.

In particular, it relates to a method for increasing the ability of a T cell expressing a cell-surface antibody to recognise low concentrations of target antigen.

Antigen recognition may be monitored by looking at T cell activation. So a T cell with increased sensitivity will be activated by a lower concentration of antigen.

T cell activation may be monitored by looking at one of the signalling events in T cell activation, such as ERK phosphorylation. Alternatively one may look at an antigen-specific effector function, such as CD107 upregulation, cytokine production or cytotoxicity.

The method of the present invention increases the sensitivity of a T cell expressing a cell-surface antibody, by lowering the affinity of the antibody to the target antigen. An increase in sensitivity may be determined by comparing the concentration of antigen necessary for T cell activation with the starting high-affinity antibody, with the concentration of antigen necessary for T cell activation with the affinity-lowered antibody. Alternatively, an increase in sensitivity may be determined by comparing the level of T cell activation achieved at a given (low) antigen concentration a) with the starting high-affinity antibody, and b) with the affinity-lowered antibody.

The sensitivity of a T cell expressing an affinity-lowered antibody may, for example, be 10, 50 or 100-fold higher than the sensitivity of a T cell expressing the equivalent natural affinity antibody.

Without wishing to be bound by theory, the present inventors predict that T cells expressing lower-affinity antibodies have higher sensitivity because they are better suited to T cell activation by serial triggering than T cells expressing high affinity antibodies.

In the serial triggering model of T cell activation, one cell-surface antibody sequentially interacts with target antigen a plurality of times, leading to a plurality of triggering events. In this model, dissociation of the antibody from the antigen is an essential step. Antibodies with low affinity and short dissociation half lives (t_{1/2}) will therefore be better suited to serial triggering than antibodies with high affinity and long t_{1/2}.

### T-CELL

The present description relates to a T cell.

The T cell is engineered to express a cell-surface antibody.

The T cell may, for example, be a cytotoxic T cell (CD8+), a helper T cell (CD4+), a regulatory T cell, or a Th17 T cell.

The T cell may be from a T cell line or a primary T cell. Primary T cells are thought to have increased peptide sensitivity compared to hybridoma cells expressing the same TCR.

### ANTIBODY

As used herein, "antibody" includes a whole immunoglobulin molecule or a part thereof, or a bioisostere, mimetic or derivative thereof. Examples of antibody fragments include: Fab, F(ab)'₂, and Fv. Examples of a bioisostere include single chain Fv (ScFv) fragments, chimeric antibodies, bifunctional antibodies.

The term "antibody" also includes monobodies, diabodies and triabodies, minibodies and single domain antibodies.

The starting antibody may be an antibody which has previously been expressed at the cell-surface of a T cell, for example as part of a chimeric antigen receptor (CAR - see below).

### CELL-SURFACE ANTIBODY

The antibody is expressed at the surface of a T cell, so may be associated with a domain that enables this expression, such as a transmembrane domain. The antibody may be expressed as a fusion protein with a transmembrane domain, such that it is expressed at the T cell surface.

### CHIMERIC ANTIGEN RECEPTORS

Chimeric antigen receptors comprise an extracellular binding domain, transmembrane domain and a cytoplasmic signalling domain. They may also comprise an extracellular hinge and spacer element.

The binding domain may comprise or consist of an antibody, such as an scFv.

An scFv commonly comprises the light (VL) and heavy (VH) variable regions of an antibody joined by a flexible linker.

ScFvs against tumour associated antigens (TAAs) have been used to produce CARs to redirect T cells against TAAs expressed at the surface of tumour cells from various malignancies including leukaemia, lymphomas and solid tumours. A major advantage of endowing T cells with non-MHC-restricted, antibody-derived specificity is that the potential target structures are no longer restricted to protein-derived peptides, but rather comprise every surface molecule on tumour cells including proteins with varying glycosylation patterns and non-protein structures such as gangliosides or carbohydrate antigens. Thus, the panel of potential tumor-specific targets is enlarged.

Other binding moieties than scFvs have also been introduced into CARs and successfully used for predefined targeting of lymphocytes. For example, chimeric receptors incorporating receptor ligands like a vascular endothelial growth factor polypeptide, an integrin-binding peptide, heregulin, or an IL-13 mutein have been explored and tumor regression was demonstrated in mouse models. Another group has successfully retargeted murine and human lymphocytes against hematopoetic tumors using a chimeric NKG2D receptor fused to the CD3 chain.

Most CARs comprise a hinge region between the binding and the transmembrane domain. The rationale for including a spacer region is to provide more flexibility and accessibility for the binding moiety, which might be otherwise buried by the dense glycocalyx shell covering T cells. Examples of extracellular spacer regions include immunoglobulin domains like the Fc regions of antibodies or immunoglobulin-like domains derived from the extracellular portions of CD8*α*, CD28, TCR*β* chain, or NKG2D.

The transmembrane region of CARs may be derived from homo- or heterodimeric type I membrane proteins like CD4, CD8, CD28, CD3, or Fc gamma.

Much attention has been paid to the intracellular signaling domains of chimeric receptors. The first generation of CARs only contains a single signaling unit, in most cases derived from the CD3 chain. Second generation CARs contain an additional costimulatory domain (CM I), predominantly the CD28 domain. Signaling through this costimulatory domain leads to enhanced proliferation, cytokine secretion, and renders engrafted T cells resistant to immunosuppression and induction of AICD. Recent developments fused the intracellular part of a second costimulatory molecule (CM II) in addition to CD28 and ITAM-bearing signaling chains, thus generating tripartite signaling CARs. T cells engrafted with third generation CARs have been reported to have superior qualities regarding effector functions and *in vivo* persistence.

### MUTAGENESIS AND SELECTION

The method of the present invention involves lowering the affinity of an antibody for its target antigen.

Techniques for altering the affinity of antibodies are known in the art. For example, mutations may be introduced into the antibody-encoding gene, and the resulting variant antibodies screened for low-affinity binders, by a technique such as yeast display or phage display.

The mutation step may be random, or targeted to specific residues in the antigen binding pocket.

The process may involve successive rounds of mutagenesis and screening, for example as part of an *in vitro* evolution process.

### ANTIGEN

The term "antigen" in terms of target antigen means an entity which is recognised (i.e. binds specifically) to the antibody expressed at the T cell surface.

An "epitope" is the portion of a molecule which is recognised by antibody. In the sense of the present invention, an antigen is or comprises at least one epitope.

An antigen may be a complete molecule, or a fragment thereof. The antigen may be or be derivable from a naturally occurring molecule.

The antigen may be or be derivable from, for example, a protein, glycoprotein, glycolipid, or carbohydrate.

Where the gene modified T cell is for use in the treatment of cancer, the antibody may recognise an antigen that is or is part of a tumour associated antigen (TAA).

Some known TAAs which are recognised by antibodies are listed in Table 1.

The T cell described herein may express a cell-surface antibody which recognises an epitope from an antigen listed in Table 1.

Where the gene modified T cell is for use in the treatment of chronic infection, the antibody may recognise an antigen that is or is part of a virus associated antigen.

Where the gene modified Treg cell is for use in the treatment of autoimmunity, the antibody may recognise a tissue-specific antigen that is expressed in the tissue affected by autoimmunity.

**Table 1**

| Antigen category | Antigen name | Tumour types |
|---|---|---|
| Haematopoietic | CD5 | T-cell leukaemia/lymphoma |
| differentiation | CD19, CD20, CD21, CD25, CD37 | B-cell lymphoma |
| antigens | CD30 | Hodgkin lymphoma |
| | CD33, CD45 | Acute myeloblastic leukaemia |
| | CAMPATH-1 (CDw52) | Lymphoid malignancies |
| | HLA-DR | Epithelial tumours (breast, colon, lung) |
| | Anti-idiotype | Epithelial tumours (breast, colon, lung) |
| Glycoproteins | Carinoembryonic antigen (CEA) | Ovarian tumours |
| | TAG-72, Ep-CAM, MUC1 | Colorectal carcinoma |
| | Folate-binding protein | Renal carcinoma |
| | A33 | Prostate carcinoma |
| | G250 | |
| | Prostate-specific membrane antigen (PSMA), Prostate specific antigen (PSA) | |
| | Ferritin | Hodgkin disease, hepatoma |
| Glycolipids | Gangliosides (e.g. GD2, GD3, GM2) | Neuroectodermal tumors, some epithelial tumours |
| Carbohydrates | Le^{y} | Epithelial tumours (breast, colon, lung, prostate) |
| | CA-125 | |
| | CA19-9 | Ovarian carcinoma |
| Growth factor | Epidermal growth factor receptor (EGFR) | Epithelial tumours |
| receptors | p185^{HER2} | Lung, glioma, breast, head and neck tumours |
| | IL-2 receptor | Breast, ovarian tumours |
| Mutated gene | De2-7 EGFR | T- and B-cell neoplasms |
| products | | Glioblastoma multiforme, breast, lung cancer |
| Stromal or vascular | Fibroblast activation protein (FAP) | Epithelial tumours (colon, breast, lung, head and neck) |
| antigens | Tenascin, metalloproteinases | Glioblastoma multiforme, epithelial tumours |
| | Endosialin, Vascular endothelial growth factor (VEGF), αᵥβ₃ | Tumour vasculature |

### AFFINITY

Affinity may be expressed in terms of the dissociation constant (K_{D}).

The natural affinity of antibodies is usually in the range 10nM - 10pM (10⁻⁸ - 10⁻¹¹ M).
TCR: MCH/peptide interactions are commonly in the range 100 - 1 µM (10⁻⁴ - 10⁻⁶ M).

The affinity of the antibody may be lowered at least 100-fold, at least 1000-fold or at least 10,000-fold.

The affinity of the antibody may be lowered such that it has an affinity for antigen which is similar to the affinity a TCR has for a MHC/peptide complex.

The T cell described herein may comprise an antibody or antibody domain having an affinity for target antigen in the range 100nm - 1 µM.

Affinity may alternatively be expressed in terms of the dissociation half time (t_{1/2}).

The present inventors have shown that increasing the half time from 9.3 seconds to 348, 1320 and 3120 seconds resulted in a loss of sensitivity.

The dissociation half time of the antibody described herein may be less than 100 seconds, or less than a minute. It may be in the range 5 to 60 seconds. It may be in the range 10-30 seconds.

### NUCLEIC ACID

The present description relates to a nucleic acid sequence encoding a cell-surface antibody as described above.

The nucleic acid sequence may be an RNA or DNA sequence or a variant thereof.

The nucleic acid sequence may encode a CAR according to the present description. In this respect, the nucleic acid sequence may comprise a sequence encoding an antibody domain operably linked to a sequence encoding a signalling domain.

The nucleic acid sequence may also comprise a nucleic acid sequence encoding a hinge region; a nucleic acid sequence encoding a spacer; and/or a nucleic acid sequence encoding a transmembrane region.

Where the nucleic acid sequence encodes a plurality of distinct sequences, such as VL and VH antibody domains, or cytoplasmic signalling domains; the nucleic acid sequence may comprise a plurality of separate sequences; a single sequence capable of producing more than one product (e.g. joined by an IRES); or a single sequence capable of producing a fused product (e.g. an scFv).

### VECTOR

The present description relates to a vector. The vector may comprise the nucleic acid sequence of the present description. The vector may be capable of expressing an antibody as defined herein in a T cell.

The vector may express the antibody at the cell-surface of the T cell.

The vector may comprise a nucleic acid sequence encoding the antibody and a transmembrane region.

The vector may be capable of expressing a chimeric antigen receptor, which comprises an antibody domain which is expressed at the T cell surface and a cytoplasmic signalling domain.

The vector may be any agent capable of delivering or maintaining nucleic acid in a host cell, and includes viral vectors, plasmids, naked nucleic acids, nucleic acids complexed with polypeptide or other molecules and nucleic acids immobilised onto solid phase particles.

The antibody is "expressed" in the host T cell by being produced as a result of translation, and optionally transcription, of the nucleic acid. Thus, the antibody is produced *in situ* in the cell prior to display at the cell surface.

Non-viral vectors may be introduced into T cells using a variety of non-viral techniques known in the art, such as transfection, transformation, electroporation and biolistic transformation.

Alternatively, the vector may be introduced into a T cell using a viral method, for example infection with a recombinant viral vector such as one derived from a retrovirus, lentivirus, herpes simplex virus or adenovirus.

Retroviral vectors and plasmids have both previously been used to express CARs in T cells.

T cells may be transfected or transformed with vector *in vitro, ex vivo* or *in vivo.*

### THERAPEUTIC METHOD

The vectors and/or antibody-expressing T cells described herein may be used to treat and/or prevent a disease.

To "treat" means to administer the vector/cell to a subject having an existing disease in order to lessen, reduce or improve at least one symptom associated with the disease and/or to slow down, reduce or block the progression of the disease.

To "prevent" means to administer the vector/cell to a subject who has not yet contracted the disease and/or who is not showing any symptoms of the disease to prevent or impair the cause of the disease (e.g. infection) or to reduce or prevent development of at least one symptom associated with the disease.

The vector/T cell described herein may be used to treat and/or prevent cancer, an infectious disease or an autoimmune disease in a subject.

The vector may be administered directly to the subject, or may be administered in vitro to T cells. In an ex vivo approach, T cells may be isolated from the subject, treated with vector such that they express low-affinity antibody, and then returned to the subject.

T cells expressing antibodies against TAAs can recognize tumor cells by binding of the CAR to a TAA. As a result, T cells are activated and can eliminate tumor cells by the secretion of perforin and granzymes as well as the expression of FasL and tumor necrosis factor-related apoptosis inducing ligand (TRAIL). In addition, other tumor-infiltrating immune cells can be activated by the secretion of various cytokines.

Several clinical trials have been conducted or are in progress using CAR-modified T cells to treat cancer patients, including the following:
(i) the use of T cells expressing a CAR recognizing carbonic anhydrase IX, to treat metastatic renal cell carcinoma (RCC);
(ii) the use of T cells expressing an *α*-folate receptor-specific CAR to treat metastatic ovarian cancer
(iii) the use of T cells expressing a L1-cell adhesion molecule to treat neuroblastoma patients
(iv) the use of T cells expressing a CD20-specific CAR to treat B-cell lymphoma or mantle cell lymphoma; and
(v) the use of EBV-specific T cells engineered with a CAR recognizing the diasialoganglioside GD2 to treat neuroblastoma.

The invention will now be further described by way of Examples, which are meant to serve to assist one of ordinary skill in the art in carrying out the invention and are not intended in any way to limit the scope of the invention.

### EXAMPLES

### Example 1 - Human T cells expressing affinity matured TCR fail to recognise Low density of MHC/peptide antigen

Antigen recognition by T cells is via the T cell receptor (TCR) that binds peptides displayed by MHC molecules. Compared to antibodies the affinity of TCR for MHC/peptide is relatively low, with dissociation constants K_{D} ranging from 100 - 1 µM. Unlike antibodies, TCR do not naturally undergo somatic hypermutation to enhance the affinity for the TCR recognised antigen.

The present inventors explored in detail how TCR/antigen affinities spanning the natural range and extending 700-fold into the supra-physiologic range affected the kinetic and the sensitivity of primary human CD8+ T cell responses. They used variants of an HLA-A0201-restricted TCR specific for a peptide derived from the Tax antigen of Human T Lymphotopic virus I (HTLV1). The affinity matured variants were generated by phage display and they retained peptide-specificity when introduced into primary CD8+ T cells. The functional analysis of transduced T cells revealed that although affinity matured TCR mediated faster responses than wild type TCR, this, paradoxically, led to an inability to recognise low concentration of antigen. The data strongly suggest that affinity maturation beyond the physiologic range accelerates human CD8+ T cell responses but disrupts the serial TCR triggering that is required to maximise recognition of low density peptide ligands.

### Results

The HLA-A0201-restricted TCR specific for a peptide (pTax) derived from the Tax protein of HTLV-1 was used in this study. Recently, phage display technology was employed to isolate affinity-matured versions of the Tax-TCR. Soluble TCR molecules and HLA/peptide complexes were analysed in the surface plasmon resonance system to measure the dissociation constant K_{D} of the TCR-HLA/peptide binding and the half-life t_{1/2} of the interaction. The binding of the wild type Tax-TCR to HLA/pTax had a K_{D} of 1.8 µM and a t_{1/2} of 9.3 seconds. The t_{1/2} of the three affinity matured versions M1-TCR, M2-TCR and M3-TCR increased to 348 sec, 1320 sec and 3120 sec, respectively, with a K_{D} for the M3-TCR of 2.5 nM (Fig. 1A). Thus, the binding of the M3-TCR to HLA/pTax was approximately 700-fold stronger and 300-fold longer than the binding of the Tax-TCR. Both the M3-TCR and the Tax-TCR bind the modified peptide pHuD with a K_{D} of 21 µM and 123 µM, respectively. Together, the TCR molecules interacting with pTax and pHuD covered the physiologic affinity range used by naturally selected TCR, and also extended into the supra-physiologic range of affinity matured TCR (Fig. 1A).

### Expression of retroviral TCR constructs in Jurkat and primary human T cells:

Human Jurkat cells and primary peripheral blood T cells were transduced with retroviral constructs encoding the Tax-TCR or the affinity matured M1, M2 and M3-TCR. Jurkat cells were stained with anti-CD3 and Vβ13 (the beta variable region segment which is used by the Tax-TCR) Abs, whilst T cells were stained with anti-CD3 and anti-CD8 and either anti-Vβ13 or HLA-A2/pTax tetramer. TCR constructs were readily expressed in human Jurkat cells (Fig. 1B). Mock-transduced T cells showed background levels of endogenous Vβ13 expression, but did not stain with HLA-A2/pTax tetramer (Fig. 1C). T cells transduced with the Tax-TCR and affinity matured TCR showed comparable percentages of CD8+ T cells and CD8- T cells expressing the Vβ13 TCR chain and capable of binding to the HLA-A2/Tax tetramer (Fig. 1C). These results indicate that affinity maturation of the TCR did not affect the transduction efficiencies or the binding of the Vβ13 antibodies and tetramer to the expressed TCR molecules.

**Kinetic of antigen-specific TCR down-regulation.** The present inventors performed experiments to test whether TCR affinity affected the kinetic of peptide-specific immune responses. In these experiments saturating concentrations of peptide were used to provide optimal TCR stimulation over defined periods of time.

As expected, stimulation of TCR-transduced bulk Jurkat T cells with the pTax peptide resulted in downmodulation of the Tax-TCR and the affinity matured versions. However, there was a dramatic difference in the speed of TCR downmodulation. Within 5 minutes of peptide stimulation the affinity matured M3-TCR showed a substantial reduction of TCR-positive T cells from 55% to 20% (Fig 2A). In addition, the 20% TCR positive T cells expressed substantially lower levels of TCR than the TCR-positive T cells prior to peptide stimulation. After 4 hours of peptide stimulation the M3-TCR was strongly downmodulated on all T cells. In contrast, the Tax-TCR displayed a much more subtle reduction in TCR expression over the 4 hour assay period. The level of peptide-driven reduction of TCR expression seen with the M1 and M2 TCR was between the slow reduction of the Tax-TCR and the fast reduction of the M3-TCR. The Tax-TCR and the M1, M2 and M3-TCR showed similar differences in the kinetic of downmodulation when the analysis was performed with transduced Jurkat cells or primary human T cells (compare Fig. 2B and C).

**Kinetic of antigen-specific signalling.** Next, the present inventors explored whether the kinetic of early signalling is different for the Tax-TCR and the affinity matured variants. For these experiments transduced primary human T cells were stimulated with saturating concentrations of HLA-A2/pTax tetramers for short time periods followed by intracellular staining for ERK phosphorylation, an early signalling event in T cell activation. Tetramers were used to achieve uniform TCR stimulation within a short time period, which was difficult to achieve with peptide loaded T2 cells. The transduced T cell populations used in these experiments contained similar numbers of TCR-positive T cells, as determined by the percentage of tetramer-positive cells (Tax-TCR = 83%, M1-TCR = 88%, M2-TCR = 92% and M3-TCR = 94%). Un-stimulated T cells showed little background phospo-ERK staining (see time point 0 in Fig. 3A). After 30 seconds of tetramer stimulation, cells expressing the affinity matured TCR showed a greater number of phospo-ERK positive cells (MI-TCR = 29%, M2-TCR = 30% and M3-TCR = 39%) compared to cells expressing Tax-TCR (13%; Fig. 3A). After 1 minute of tetramer activation the Tax-TCR reached 30% phospo-ERK positive T cells, which is the response rate seen by the M1 and M2-TCR cells after 0.5 minutes. At the 1 minute time point the response by the M1, M2 and M3 TCR had increased to 49%, 50% and 56%, respectively. After a peak of ERK phosporylation the signal deceased and returned to nearly baseline after 10 minutes (Fig. 3A). In addition to the increased percentage of responding T cells at each time point, the intensity of ERK phosphorylation in the responding cells was greater for the affinity matured TCR compared to the Tax-TCR (Fig. 3B). Together, these data showed that affinity matured TCR triggered faster and more potent phospo-ERK signals in the early phase of T cell stimulation.

**Kinetic of antigen-specific CD107 up-regulation.** In order to determine if the faster signalling and TCR down-regulation of the affinity matured TCR correlated with accelerated antigen-specific effector function, the present inventors investigated the upregulation of CD107, a molecule that is exposed on the surface of T cells releasing effector molecules such as perforin and cytokines from intracellular granules. Primary T cells expressing similar levels of the Tax-TCR or the affinity matured variants were stimulated with antigen-presenting T2 cells loaded with saturating concentrations of pTax peptide. Over a two-hour stimulation period, all TCR transduced populations showed an increase in the percentage of CD107 positive cells (Fig. 4A). However, the CD107 upregulation for the Tax-TCR was substantially slower than upregulation observed for the M3-TCR. At 2 hours the number of CD107 positive cells expressing the Tax-TCR was similar to the number seen for the M3-TCR at 0.5 hours. The speed of CD107 upregulation for the M1 and M2-TCR was between the Tax-TCR and M3-TCR, indicating a correlation between increasing half-life of TCR-antigen binding and accelerated kinetic of CD107 upregulation.

In order to analyse the kinetics of CD107 upregulation within the natural affinity range, primary T cells expressing the Tax-TCR or the M3-TCR were stimulated with T2 cells coated with saturating concentrations of the pHuD peptide. As before, the fastest response was seen when the M3-TCR was stimulated with the pTax peptide, followed by Tax-TCR stimulation with pTax peptide. The response of M3-TCR stimulated with pHuD was approximately 2 hours slower than the same TCR stimulated with pTax. The response of the Tax-TCR stimulated by pHuD was approximately 4 hours slower compared to stimulation with pTax (Fig 4B).

**Affinity matured TCR fail to respond to low concentration of antigen:** The present inventors next explored whether the accelerated response displayed by affinity matured TCR correlated with improved performance at low concentrations of peptide antigen. To explore this, transduced T cells were stimulated with decreasing peptide concentrations for 18 hours, a time period sufficient to initiate responses irrespective of the kinetic of the individual TCR/antigen combinations.

The peptide sensitivity of the CD107 response was assessed using transduced T cells expressing similar levels of the Tax-TCR or the affinity matured versions. The T cells were stimulated with T2 cells loaded with decreasing concentrations of the pTax or pHuD peptide. The low affinity Tax-TCR/pHuD combination required approximately 10-fold more peptide to trigger a CD107 response than the medium affinity M3-TCR/pHuD combination (Fig. 4C). The best peptide sensitivity was achieved by the Tax-TCR/pTax combination with a K_{D} at the upper end of the natural affinity range. However, a further 700-fold increase in affinity above the natural range did not enhance the functional sensitivity of T cells but instead abolished the ability to respond to the same low peptide concentrations that were recognised by the Tax-TCR (Fig. 4C). The antigen concentrations required to trigger CD107 upregulation in T cells expressing the M3-TCR was approximately 100-fold higher compared to the response by the Tax-TCR. Similarly, the M1 and M2-TCR were less sensitive than the Tax-TCR, but more sensitive than the M3-TCR. This indicated that a progressive increase in TCR affinity above the natural range resulted in a progressive inability to recognise low density of HLA/peptide.

The present inventors used IFNγ production to explore if the unexpected loss in T cell sensitivity was peculiar to CD107 upregulation or also affected other T cells effector functions. Again, T cells expressing the Tax-TCR produced IFNγ at lower concentrations of pTax peptide than T cells expressing the affinity matured TCR (Fig. 5A). Similar to the CD107 profile, the M3-TCR displayed lower sensitivity than the M1 and M2-TCR. However, the poor performance of the M3-TCR compared to the Tax-TCR was reversed when T cells were stimulated with the low affinity pHuD ligand. In this situation the M3-TCR performed approximately 10-fold better than the Tax-TCR (Fig. 5A), indicating that poor recognition of low density antigen was linked to the high affinity M3-TCR/pTax combinations and was not an inherent feature of the affinity matured M3-TCR.

Finally, the present inventors determined the peptide concentrations required to mediate TCR down-regulation. Although the speed of down-regulation was much faster for the affinity matured TCR (see Fig. 2), peptide titration clearly revealed that down-regulation of the Tax-TCR required less peptide than down-regulation of the M1, M2 and M3-TCR (Fig. 5B). Consistent with the results of the IFNγ and CD107 response, 100-fold more peptide was required for down-regulation of the M3-TCR compared to the Tax-TCR. Together, this indicates that the improved speed of affinity matured TCR was linked to a failure to recognise low concentrations of antigen.

In the final experiments the present inventors explored the ability of the Tax-TCR and the affinity matured TCR to recognise naturally processed Tax antigen. HLA-A2 positive HeLa cells were transfected with a plasmid vector encoding full length Tax protein and GFP as marker, or with a control vector encoding GFP only. The transfected HeLa cells were used to stimulate T cells expressing the Tax-TCR or affinity matured TCR followed by measurement of Tax-specific CD107 induction. A robust CD107 up-regulation was seen when T cells expressing the Tax-TCR and the M1-TCR were stimulated with the Tax-expressing HeLa cells (Fig 5C). In contrast, impaired CD107 responses were seen when T cells expressing the M2 and M3-TCR were analysed; M3-TCR T cells showed only 50% of the response seen with Tax-TCR T cells. Stimulation with control GFP transfected HeLa cells showed similar low background levels of CD107 for all TCR, excluding non-specific T cell stimulation by transfected HeLa cells. Finally, stimulation with peptide loaded HeLa cells triggered equally strong CD107 responses in T cells expressing Tax-TCR or affinity matured M1, M2 or M3-TCR, indicating that all T cells were fully functional when stimulated with high concentration of peptide antigen. Together, these experiments indicated that the reduced ability of the M2 and M3-TCR to recognise low concentration of peptide antigen (Fig 4C) correlated with an impaired ability to recognise cells expressing the Tax antigen endogenously (Fig 5C).

### Discussion

The data presented here show that within the 'natural' TCR affinity range there is a good correlation between dissociation constant K_{D} of the TCR/ligand interaction and the speed and peptide sensitivity of T cell responses. However, beyond the 'natural' affinity range there is a separation of the speed and the sensitivity of CD8+ T cell responses. Whilst affinity matured TCR trigger faster responses, this was linked to a reduction in peptide sensitivity. Optimal peptide sensitivity was achieved with the Tax-TCR (t_{1/2} 9.3 sec) and increasing the dissociation half time t_{1/2} to 348, 1320 and 3120 seconds resulted in a progressive loss of peptide sensitivity. A recent study with the human NY-ESO-TCR showed that an increase in t_{1/2} from 2.2 sec for the wild type TCR to 19 sec for an affinity matured TCR improved the ability of TCR transduced CD8+ T cells to recognise NY-ESO expressing tumour cells, while this improvement was not seen with an affinity matured TCR with a t_{1/2} of 41 sec (Robbins et al. (2008) J Immunol. 180:6116-6131). Together, these observations suggest that affinity maturation may be beneficial when the half-life of the TCR-HLA/peptide interaction is increased to >10 sec, but is likely to impair peptide sensitivity when the t_{1/2} is enhanced into the range of minutes.

The presented data support the serial triggering model of T cell activation, which predicts that one MHC/peptide complex can sequentially engage several TCR molecules to achieve a critical threshold of approximately 200 triggered TCR that is required to achieve T cell activation. In this model, the dissociation of the TCR is an essential step, which is progressively impaired with increasing the K_{D} and half-live of the TCR-MHC/peptide complex. The experimental data presented here are fully compatible with the serial triggering model and provide an explanation for the relative low affinity and short t_{1/2} of the natural TCR repertoire. Our data show that affinity matured TCR do not improve the ability of T cells to recognise small numbers of MHC presented peptide epitopes, an essential function of high avidity T cells. The ability of T cells to recognise low concentration of peptide was found to correlate with an improved ability to control the growth of tumor cells and the spread of virus infection in vivo, supporting the biological importance of high T cell avidity (Alexander-Miller et al (1996) Proc Natl Acad Sci USA. 93:4102-4107; Zeh et al (1999) J Immunol. 162:989-994). Similarly, the present inventors recently demonstrated a correlation between the functional avidity of Tax-specific CTL, assayed directly ex vivo, and the efficiency of control of HTLV-1 replication in vivo (Kattan et al (2009) J Immunol. 182:5723-5729).

### Materials and Methods

### Media, cells, antibodies, tetramer and peptides:

Unless otherwise stated all culture medium was RPMI (Cambrex, UK) supplemented with 10% heat inactivated FCS (Sigma, UK), 1% penicillin / streptomycin (Invitrogen, UK) and 1% L-glutamine (Invitrogen). Cell lines used were the HLA-A2 positive T2 cell line that is deficient in TAP (transporter associated with antigen processing) and can be efficiently loaded with exogenous peptides and HeLa cells previously stably transduced to express HLA-A2. HLA-A2 positive peripheral blood mononuclear cells (PBMCs) were obtained from volunteer donors from the National Blood Service, Collindale, London, UK. Flow cytometry antibodies were anti-human PE CD107a, PerCP CD3 and APC CD8 (BD Biosciences, UK), PE Vβ13.1 (Immunotech, UK), phosphor-p44/42 MAPK (ERK1/2) (Thr202/Tyr204) (197G2) rabbit mAb (Cell Signaling Technology, UK) and Alexa Fluor 488 F(ab)2 fragment of goat anti-rabbit IgG (Invitrogen, UK). PE-labelled HLA-A2/Tax tetramers were obtained from Beckman Coulter. The peptides used in this study were the HLA-A2 binding peptides pTax (LLFGYPVYV), pHuD (LGYGFVNYI) and pWT235 (CMTWNQMNL) and were synthesised by ProImmune (Oxford, UK).

### Generation of wild-type and mutated Retroviral TCR constructs:

Only the beta TCR chain underwent affinity maturation and was accomplished as detailed previously by Li et al (2005). The TCR alpha and beta chain constructs were cloned together into retroviral pMP71 vectors and were linked via a viral p2A sequence. The alpha and beta TCRs contained an additional inter-chain disulphide bound in the constant domains. This was achieved by replacing residue 48 of the alpha chain from a threonine to a cysteine and residue 57 of the beta chain from a serine to a cysteine as previously described (REF). The TCR construct was codon optimised.

### Transduction of retroviral TCR constructs into Jurkat cells and primary T cells:

For retroviral transduction, 2 x 10⁶ phoenix amphotropic packaging cells were cultured in 10 cm culture plates for 24 h at 37 °C, 5% CO₂ in DMEM media supplemented with 10% heat-inactivated FCS, 1% penicillin / streptomycin and 1% L-glutamine. The phoenix amphotropic packaging cells were cultured in fresh DMEM media and transfected with 16 µg of the vector constructs using calcium phosphate precipitation (Invitrogen). 24 h after transfection the media was replaced with RPMI culture media and incubated for a further 24 h. The viral supernate was then harvested. Jurkat cells were split 24 h prior to retroviral transduction and PBMNs were activated for 48 h using the anti-CD3 antibody OKT3 at 30 ng/ml and interleukin (IL)-2 at 600 U/ml (Chiron, CA). Retroviral transductions were conducted on retronectin (Takara, Japan) coated 24 well plates. 1 ml of virus supernate was added per well and the plates was spun at 2,000g, 32°C, 2 h. Wells were washed twice with PBS and cells were seeded at 5 x 10⁵ per well in 2 ml of culture media. PBMC were transduced in the presence of IL-2 at 600 U/ml. After 24 h of incubation, the media was replaced with fresh culture media. Transduction efficiency was determined by flow cytometry analysis, conducted on a LSR II flow cytometer after a further 48 h culture period. FACS data was analysed using FACSdiva.

### Antigen-stimulation and expansions of TCR transduced T cells:

TCR transduced primary T cells were stimulated and expanded every 7 - 10 days. The stimulations were conducted in 24 well plates in 2 ml culture media containing 10 % non-heat inactivated FCS and 10 U/ml IL-2 (Roche, UK), at 37 °C, 5 % CO₂. Each well contained, 5 x 10⁵ transduced cells, 2 x 10⁵ irradiated T2 cells loaded for 2 h with 100 µM of the peptide pTax and 2 x 10⁶ irradiated autologous PBMCs as feeder cells.

### TCR down-negulation assay:

2 x 10⁵ TCR transduced T cells or Jurkat cells were co-cultured with 2 x 10⁵ T2 cells pre-loaded with peptide in round-bottom 96 well plates in 200 µl culture media. After each co-culturing time point the reaction was immediately stopped by washing in ice-cold PBS. Jurkat cells were stained with anti-Vβ13.1 Abs and T cells were stained with anti-CD8 Abs and tetramer and FACs analysed.

### Phospho-ERK assays:

2 x 10⁵ TCR transduced T cells were cultured with 1 in 500 dilution of tetramer in 100 µl of culture media for the stated times. After each time point the reaction was immediately stopped by adding 2% paraformaldehyde for 10 min at 37°C. Cells were washed, permeabilised with 90% methanol (ice, 30 min) and then stained with anti-phosphor-p44/42 MAPK, followed by Alexa Fluor 488 F(ab)2 fragment of goat anti-rabbit IgG for FACs analysis.

### CD407a up-regulation assays:

For assays using T2 cells as stimulator cells, 2 x 10⁵ T2 cells were pre-loaded with peptide. For assays using HLA-A2 positive HeLa cells as stimulator cells, the HeLa cells were transfected with either a pGFPC1 plasmid (Clontech) or a full length Tax gene encoded within the pGFPC1 plasmid³⁰. Cells were transfected using 8µg plasmid DNA and 24 µl Fugene (Roche, UK). 24 h after transfection the GFP expression was determined and was typically 20 - 25%. For stimulations, the ratio of TCR-positive T cells to GFP positive HeLa cells was 1:1. As positive control TCR-positive T cells were stimulated with transfected HeLa cells loaded with saturating concentration of pTax peptide (100µM) at a ratio of 1:1. T2 cells or HeLa cells were co-cultured with 2 x 10⁵ TCR transduced T cells in round-bottom 96 well plates in 250 µl culture media containing 1µg/ml brefeldin A (Sigma, UK), 0.7µg/ml BD GolgiStop (BD Bioscience Pharmingen, UK) and 2 µl of CD107a PE. After each co-culturing time point the reaction was immediately stopped by washing in ice-cold PBS. T cells were stained with anti-CD8 and FACS analysed.

### ELISA:

For cytokine secretion assays, 1 x 10⁵ TCR transduced T cells were stimulated with 1 x 10⁵ irradiated T2 cells loaded for 2 h with peptide. Assays were conducted in triplicates in round-bottom 96 well plates in 250 µl culture media. After 18 h incubation at 37 °C, 5 % CO₂, supernatant was harvested and tested for secreted IFNγ using a human enzyme linked immunosorbent assay (ELISA) kit (BD Biosciences) as per the manufacturer's instructions. The data was analysed using excel software.

## Claims

1. A method for increasing the sensitivity of a T cell expressing a cell-surface antibody to a target antigen, which comprises the step of lowering the affinity of the antibody to the target antigen by at least 100-fold, wherein the affinity of the antibody is lowered by mutagenesis of the antibody, followed by *in vitro* selection for low affinity variants.

2. A method according to claim 1, wherein the affinity of the antibody to the target antigen is lowered by at least 1000-fold.

3. A method according to any preceding claim, wherein the affinity of the antibody for the target antigen, following lowering, is in the range of 100 - 1µM.

4. A method according to any preceding claim, wherein the antibody, following affinity lowering, has a dissociation half time from the target antigen in the range of 5 - 60 seconds.

5. A method according to any preceding claim, wherein the antibody is part of a chimeric antigen receptor (CAR) comprising:
(i) an antibody domain, expressed at the T cell surface; and
(ii) a cytoplasmic signalling domain.

## Patentansprüche

1. Verfahren zur Steigerung der Empfindlichkeit einer einen Zelloberflächenantikörper gegen ein Zielantigen exprimierenden T-Zelle, das den Schritt der Verringerung der Affinität des Antikörpers für das Zielantigen um wenigstens das 100-Fache, wobei die Affinität des Antikörpers durch Mutagenese des Antikörpers verringert wird, gefolgt von einer Invitro-Selektion auf niedrigaffine Varianten umfasst.

2. Verfahren nach Anspruch 1, wobei die Affinität des Antikörpers für das Zielantigen um wenigstens das 1000-Fache verringert wird.

3. Verfahren nach einem vorhergehenden Anspruch, wobei die Affinität des Antikörpers für das Zielantigen nach dem Verringern im Bereich von 100 - 1 µM liegt.

4. Verfahren nach einem vorhergehenden Anspruch, wobei der Antikörper nach Affinitätsverringerung eine Dissoziationshalbwertszeit vom Zielantigen im Bereich von 5 - 60 Sekunden aufweist.

5. Verfahren nach einem vorhergehenden Anspruch, wobei der Antikörper Teil eines chimären Antigen-Rezeptors (CAR), der
(i) eine Antikörperdomäne, exprimiert an der T-Zelloberfläche; und
(ii) eine cytoplasmatische Signalgebungsdomäne umfasst, ist.

## Revendications

1. Méthode d'augmentation de la sensibilité d'une cellule T exprimant un anticorps de surface cellulaire dirigé contre un antigène cible, qui comprend à l'étape de diminution de l'affinité de l'anticorps pour l'antigène cible d'au moins 100 fois, où l'affinité de l'anticorps est diminuée par mutagenèse de l'anticorps, suivie d'une sélection *in vitro* de variantes de faible affinité.

2. Méthode selon la revendication 1, où l'affinité de l'anticorps pour l'antigène cible est diminuée d'au moins 1000 fois.

3. Méthode selon l'une quelconque des revendications précédentes, où l'affinité de l'anticorps pour l'antigène cible, après diminution, est située dans la plage 100 - 1 µM.

4. Méthode selon l'une quelconque des revendications précédentes, où l'anticorps, après diminution de l'affinité, présente une demi-vie de dissociation de l'antigène cible dans la plage 5 - 60 secondes.

5. Méthode selon l'une quelconque des revendications précédentes, où l'anticorps est parti d'un récepteur antigène chimérique (CAR) comprenant :
(i) un domaine d'anticorps, exprimé à la surface de la cellule T ; et
(ii) un domaine de signalisation cytoplasmique.
